# EUROPEAN PATENT APPLICATION

(11) **EP 1 813 295 A2**
(43) Date of publication of application: **01.08.2007**
(21) Application number: 07001483.2
(22) Date of filing: 24.01.2007
(51) Int. Cl.: A61L 31/00

(54) **Coated surgical needles**

(30) Priority: 25.01.2006 US 339652
(71) Applicant: Tyco Healthcare Group LP, Norwalk, Connecticut 06856 (US)
(72) Inventor: Roby, Mark S., Killingworth, CT 06419 (US)
(74) Representative: HOFFMANN EITLE

(57) **Abstract**

The present disclosure relates to surgical needles having a coating thereon, the coating formed from a coating composition comprising at least one anesthetic material.

## Description

### CROSS-REFERENCE TO RELATED APPLICATIONS

This application is a continuation-in-part of co-pending U.S. Patent Application Serial No. 10/401,370, filed on March 28, 2003, which is a continuation-in-part application of U.S. Serial No. 09/964,901, filed on September 27, 2001, now U.S. Patent No. 6,936,297. The disclosure of each of these priority documents are incorporated herein in their entirety.

### BACKGROUND

### Technical Field

The present disclosure generally relates to surgical needles. More particularly, the present disclosure is directed to coated surgical needles which employ a coating composition containing at least one anesthetic agent to reduce the pain inflicted to a patient compared with the pain inflicted by a standard surgical needle upon penetration of the same or similar tissue.

### Background of Related Art

In general, the siliconization of metallic cutting edges of articles such as, for example, razor blades, hypodermic needles, scissors, scalpels, and curettes, is known. For example, Dow Coming Corporation's Dow Coming.RTM. MDX4-4159 Fluid has been used to siliconize cutting edges with an ambient temperature and humidity-curable mixture of an aminoalkyl siloxane and a cyclosiloxane dissolved in a mixture of Stoddard solvent and isopropyl alcohol.

U.S. Pat. No. 3,574,673, the contents of which are incorporated by reference herein, discloses the silicone coating of a cutting edge employing a siliconization fluid containing a mixture of copolymerizable silicones made up of an aminoalkyl siloxane, specifically a (polyaminoalkyl) alkoxysilane, and a dimethylpolysiloxane.

Other examples include U.S. Pat. Nos. 5,258,013 and 5,458,616 which disclose coating surgical needles with a siliconization material containing an aminoalkyl siloxane and a cyclosiloxane employing ultrasonic radiation. The siliconization material can be applied in a solvent carrier, e.g., hexane or heptane.

Yet another example is U.S. Pat. No. 5,985,355 which discloses coating surgical needles by (1) coating the needle with a coating solution comprising a highly condensable polydimethylsiloxane in a solvent to form a leveling coat; (2) evaporating the solvent from the first coating; (3) curing the leveling coating to polymerize the polydimethylsiloxane; (4) applying a second coating solution over the leveling coat comprising a polydimethylsiloxane having amino and alkoxy functional groups and a solvent; and (5) evaporating the solvent from the second coating.

It would be advantageous to provide coated surgical needles which inflict reduced amounts of pain upon each passage through tissue during a suturing operation.

### SUMMARY

It has been discovered that a silicone coating derived from a coating mixture comprising at least one polydialkylsiloxane having a molecular weight sufficient to provide a viscosity of the coating mixture of at least about 10,000 cp and at least one other siliconization material can be applied to a surgical needle to provide a siliconized surgical needle in which the siliconized needle exhibits an average tissue penetration force below that of a standard siliconized surgical needle. In one embodiment of the present disclosure there is provided a siliconized surgical needle obtained by applying to the surface of the needle a coating mixture comprising at least one polydialkylsiloxane having a molecular weight sufficient to provide a viscosity of the coating mixture of at least about 10,000 cp and at least one other siliconization material and thereafter curing the coating mixture to provide a copolymerized coating on the needle.

In another embodiment of the present disclosure, a siliconized surgical needle can be obtained by applying to the surface of the needle a coating mixture containing a polydialkylsiloxane and at least one siliconization material which does not covalently bond with the polydialkylsiloxane and thereafter subjecting the coating mixture to curing conditions such that the siliconization material cross-links thereby interlocking the polydialkylsiloxane in the coating to provide an interpenetrating networked coating.

The expression "standard siliconized surgical needle" or "standard needle" as used herein refers to a commercially available siliconized surgical needle, e.g., the siliconized surgical needles attached to sutures marketed by Ethicon, Inc. (Somerville, N.J.).

In embodiments, a surgical needle having a coating thereon may be formed from a coating composition comprising at least one anesthetic material.

While the amount of force required to achieve penetration of tissue during suturing may initially be about the same for the coated surgical needles of this disclosure and a standard surgical needle, and while both needles will tend to experience an increase in penetration force with each successive passage through tissue, at the conclusion of any given number of such passages, the coated needle of this disclosure will inflict less pain to the patient compared with the pain inflicted by a standard surgical needle upon penetration of the same or similar tissue.

### DESCRIPTION OF THE PREFERRED EMBODIMENTS

Siliconized surgical needles are produced in accordance with the present disclosure. By coating a surgical needle with a coating mixture containing at least one polydialkylsiloxane having a molecular weight sufficient to provide a viscosity of the coating mixture of at least about 10,000 cp and at least one siliconization material, a siliconized surgical needle is provided which exhibits a significantly reduced tissue penetrating force compared with that of a standard surgical needle after an equivalent number of passages through the same, or substantially the same, tissue. Thus, the average tissue penetration force of the siliconized needle herein will advantageously be less than about 10%, preferably less than about 20% and more preferably less than about 30%, of the average tissue penetration force of a standard siliconized needle from after about 5 to about 20 passes through the same or similar tissue.

Surgical needles which can be coated with the coating mixture in accordance with this disclosure can be manufactured from a variety of metals. Such metals include, for example, Series 400 and Series 300 stainless steels. Other suitable metals for the fabrication of surgical needles include the quaternary alloys disclosed in U.S. Pat. Nos. 3,767,385 and 3,816,920, the contents of which are incorporated by reference herein. A preferred quaternary alloy possesses the ranges of components set forth below in Table I:

**TABLE I**

| COMPOSITION OF SURGICAL NEEDLE QUATERNARY ALLOY (WT. %) | | | |
|---|---|---|---|
| Component(s) | Broad Range | Preferred Range | Most Preferred Range |
| Nickel | 10-50 | 24-45 | 30-40 |
| Cobalt | 10-50 | 25-45 | 30-40 |
| Nickel + Cobalt | 50-85 | 60-80 | 65-75 |
| Chromium | 10-30 | 12-24 | 15-22 |
| Molybdenum, tungsten and/or niobium (columbium) | 5-20 | 8-16 | 10-13 |

Another preferred quaternary alloy within Table I which can be utilized for the siliconized needle of this disclosure, designated MP35N, is available in wire form from Maryland Specialty Wire, Inc. (Cockeysville, Md.) and contains (nominal analysis by weight): nickel, 35%; cobalt, 35%; chromium, 20% and molybdenum, 10%.

In general, application of a coating mixture containing at least a polydialkylsiloxane having a molecular weight sufficient to provide a viscosity of the coating mixture of at least about 10,000 cp and at least one siliconization material to a surgical needle followed by curing will provide a siliconized surgical needle meeting the requirements of this disclosure.

Suitable polydialkylsiloxanes for use in forming the coating mixture herein include polydimethylsiloxanes, polydiethylsiloxanes, polydipropylsiloxanes, polydibutylsiloxanes and the like with polydimethylsiloxanes being preferred. Particularly preferred polydimethylsiloxanes are polydimethylsiloxanes having a molecular weight sufficient to provide a viscosity of the coating mixture of at least about 10,000 cp and preferably of at least about 30,000 cp. Such polydimethylsiloxanes for use herein are the products sold by Dow Coming under the name "Syl-Off DC 23", which is suitable as a high density condensable polydimethylsiloxane, and NuSiI Technology under the name "MED 1-4162" (30,000 cp).

Suitable siliconization materials for addition with the foregoing polydialkylsiloxanes to form the coating mixtures of this disclosure include siliconization materials containing an aminoalkyl siloxane and at least one other copolymerizable siloxane, e.g., an alkylpolysiloxane or a cyclosiloxane; a silicone oil, e.g., one sold by Dow Coming Corporation under the name Dow 36 Medical Fluid (350 to 12,500 centistokes), and the like with the siliconization material containing an aminoalkyl siloxane and at least one other copolymerizable siloxane being preferred. Generally, the preferred siliconization material includes (a) from about 5 to about 70 weight percent of an aminoalkyl siloxane of the general formula: 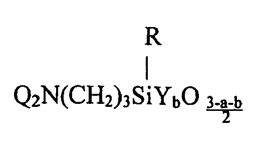 wherein R is a lower alkyl radical containing no more than about 6 carbon atoms; Y is selected from the group consisting of --OH and --OR' radicals in which R' is an alkyl radical of no more than about 3 carbon atoms; Q is selected from the group consisting of hydrogen, --CH₃ and --CH₂CH₂NH₂; a has a value of 0 or 1, b has a value of 0 or 1 and the sum of a+b has a value of 0, 1 or 2; and (b) from about 30 to about 95 weight percent of a methyl substituted siloxane of the general formula: 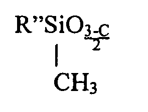 wherein R" is selected from the group consisting of --OH and --CH₃ radicals and c has a value of 1 or 2. The two components of this siliconization material copolymerize, forming a lubricating coating on the surface of the needle.

In addition to, or in lieu of, the foregoing second copolymerizable siloxane, one can use one or more cyclosiloxanes such as, e.g., those described in the "Encyclopedia of Polymer Science and Engineering", Mark et al., eds., 2nd ed., Vol. 15, John Wiley & Son (1989), p. 207 et seq., the contents of which are incorporated by reference herein, provided, of course, the total amount of the second copolymerizable siloxane(s) is within the aforestated range.

A particularly preferred siliconization material for use herein in combination with the aforementioned polydimethylsiloxane(s) to form the coating mixture is Dow Coming Corporation's Dow Coming.RTM. MDX 4-4159 Fluid ("MDX Fluid"), an active solution of dimethyl cyclosiloxanes and dimethoxysilyldimethylaminoethylaminopropyl silicone polymer in a mixture of Stoddard solvent (mineral spirits) and isopropyl alcohol. Another preferred siliconization material is NuSiI Technology's MED-4159.

In one embodiment of the present disclosure, the coating mixture can be formed by adding a first solution of at least one of the foregoing polydialkylsiloxanes in a solvent with a second solution of at least one of the foregoing siliconization materials in a solvent. Under preferred conditions, the first solution can be prepared by adding Syl-Off DC 23, MED1-4162 or both in a solvent such as, for example, a hydrocarbon solvent having from about 5 to about 10 carbon atoms, e.g., pentane, hexane, heptane, octane, etc., xylene, chlorinated solvents, THF, dioxanone and the like and mixtures thereof with hexane being preferred. The first solution is typically formed from Syl-Off DC 23 or MED1-4162 with hexane with Syl-Off DC 23 or MED 1-4162 being present in the concentration range of from about 10 g/l to about 70 g/l and preferably from about 35 g/l to about 45 g/l.

The second solution, also under preferred conditions, can be prepared in the form of a dilute organic solution, e.g., one prepared with a solvent such as, for example, a hydrocarbon solvent possessing from about 5 to about 10 carbon atoms, e.g., pentane, hexane, heptane, octane, etc., trichlorotrifluoroethane, 1,1,1-trichloroethane, mineral spirits, alcohols, e.g., isopropyl alcohol, and the like and mixtures thereof. It is preferred to dilute MDX Fluid (or other siliconization material) with hexane and isopropyl alcohol with MDX Fluid being present in the concentration range of from about 10 g/l to about 80 g/l and preferably from about 20 g/l to about 40 g/l. In a preferred embodiment, the siliconization material is a mixture of MED 1-4162 and MDX Fluid.

The mixture will ordinarily be formed by adding the first solution of the polydialkylsiloxane in solvent with the second solution of the siliconization material in solvent in a ratio ranging from about 12:1 to about 1:12, preferably from about 6:1 to about 1:6 and more preferably from about 2:1 to about 1:2. As one skilled in the art will readily appreciate, the amount of the first and second solutions necessary in forming the mixtures herein will vary depending on the volume of mixture desired.

Once the coating mixture is formed, it can then be applied to the foregoing needles employing techniques known to one skilled in the art, e.g., by dipping, wiping, spraying, total immersion, etc, with dipping and spraying being the preferred techniques. Preferably, the needles are dipped into the coating mixture for about 5 to about 60 seconds, preferably about 10 to about 45 seconds and more preferably from about 15 to 30 seconds to form a coating on the needles. After evaporation of any dilutant or solvent carrier, the siliconized coating is cured to the desired degree.

The coating can be cured by, for example, first placing the coated needle in a humid environment, e.g., a humidification chamber, and exposing the coated needle to a temperature of from about 10°C. to about 50°C. and preferably from about 20°C. to about 35°C in a relative humidity of from about 20% to about 80% and preferably from about 50% to about 65%. The coated needles are subjected to the foregoing temperatures and humidities to initiate curing to the desired degree and provide an improved lubrication coating. Typically, a time period ranging from about 1 hour to about 6 hours and preferably from about 2 hours to about 4 hours is employed. The coated needles are then placed in, e.g., furnace or oven, and cured by heating the needles to a temperature of from about 100°C to about 200°C, preferably from about 110°C to about 150°C and more preferably from about 115°C to about 150°C for a time period ranging from about 2 hours to about 48 hours and preferably from about 15 hours to about 25 hours such that cross-linking of the polydialkylsiloxane and siliconization material occurs. In a particularly useful embodiment, the coated needles are heated to a temperature of 140°C for 4 hours and a temperature of 120°C for 20 hours.

In another embodiment of the present disclosure, the coating mixture herein is formed from at least a polydialkylsiloxane and a siliconization material which does not covalently bond with the polydialkylsiloxane. A suitable polydimethylsiloxane for use herein which does not covalently bond with the siliconization material is a product sold by NuSiI Technology under the name "MED-4162". Generally, the mixture is formed by adding a first solution containing at least the polydimethylsiloxane in a solvent with the second solution discussed hereinabove. The first solution is preferably formed employing the polydimethylsiloxane MED-4162 in a solvent such as, for example, a hydrocarbon solvent having from about 5 to about 10 carbon atoms, e.g., pentane, hexane, heptane, octane, etc., xylene, and the like and mixtures thereof with hexane being preferred. It is particularly preferred to form the first solution from MED-4162 in hexane in generally the same ranges as the first solution discussed above and then adding the first solution and second solution in generally the same ratios as discussed above to form the coating mixture. Once the mixture is formed, it can then be applied to the surface of a surgical needle employing generally the same techniques and parameters as discussed above. The coating mixture is then subjected to curing conditions, e.g., the curing steps discussed above, such that the siliconization material polymerizes and cross-links thereby interlocking the polydimethylsiloxane in the coating resulting in an interpenetrating network coating.

One or more medico-surgically useful substances may be incorporated into the coating mixture, e.g., those which accelerate or beneficially modify the healing process when particles are applied to a surgical repair site. So, for example, the composition can carry a therapeutic agent which will be deposited at the repair site. The therapeutic agent can be chosen for its antimicrobial properties, capability for promoting repair or reconstruction and/or new tissue growth. Antimicrobial agents such as broad spectrum antibiotics (gentamycin, erythromycin or derivatized glycopeptides) which are slowly released into the tissue can be applied in this manner to aid in combating clinical and sub-clinical infections in a tissue repair site. Additional suitable antimicrobial agents include, .beta.-lactams, quinolones, aminoglycosides, antibiotics (such as cephalosporins, penecillins, quinolones, tetracyclines, erythromycins, extended-spectrum macrolides, aminoglycosides, sulfonamides, chloramaphenicol, clindamycin, vancomycin, spectinomycin, carbapenems, monobactams, streptogramin, fosfomycin, tromethamines and teicoplanins) fusidic acid, novobiocin, minocycline, rifampin, and polymyxin. Also suitable are biocides such as heavy metals (Ag, Zn, Cu, Ge) their salts and derivatives; biguanides (such as chlorhexidine, alexidine, and polymeric biguanides), their salts and derivatives; phenols and bisphenols (such as triclosan and hexachlorophene) their salts and derivatives; halogen releasing agents (such as iodine and iodosphers); and quatenary ammonium compounds. To promote repair and/or tissue growth, one or several growth promoting factors can be introduced into the sutures, e.g., fibroblast growth factor, bone growth factor, epidermal growth factor, platelet derived growth factor, macrophage derived growth factor, alveolar derived growth factor, monocyte derived growth factor, magainin, and so forth. Some therapeutic indications are: glycerol with tissue or kidney plasminogen activator to cause thrombosis, superoxide dimutase to scavenge tissue damaging free radicals, tumor necrosis factor for cancer therapy or colony stimulating factor and interferon, interleukin-2 or other lymphokine to enhance the immune system. The composition may also include antiseptics, anti-inflammatory agents and anesthetics.

In embodiments, the coating composition may include at least one anesthetic material. Suitable anesthetic materials include any material capable of reducing pain. Some examples include, but are not limited to, ambucaine, amolanone, amylocaine, benoxinate, benzocaine, betoxycaine, biphenamine, bupivacaine, levo-bupivacaine, butacaine, butamben, butanilicicaine, butethamine, butoxycaine, carticaine, cocaethylene, cyclomethycaine, dibucaine, dimethisoquin, dimethocaine, diperodon, dyclonine, ecgonidine, ecgonine, ethyl aminobenzoate, ethyl chloride, levo-etidocaine, etidocaine, dextro-etidocaine,.beta.-eucaine, euprocin, fenalcomine, fomocaine, hexylcaine, hydroxyprocaine, hydroxytetracaine, isobutyl p-aminobenzoate, leucinocaine mesylate, levoxadrol, lidocaine, meperidine, levo-mepivacaine, mepivacaine, meprylcaine, metabutoxycaine, methyl chloride, myrtecaine, naepaine, novacaine, octacaine, orthocaine, oxethazaine, parethoxycaine, phenacaine, phenol, a pipecoloxylidide, piperocaine, piridocaine, polidocanol, pramoxine, sameridine, prilocaine, propanocaine, proparacaine, propipocaine, propoxycaine, pseudococaine, pyrrocaine, quinine urea, risocaine, ropivacaine, levo-ropivacaine, salicyl alcohol, tetracaine, tolycaine, trimecaine, veratridine, zolamine, a 2-alkyl-2-alkylamino-2',6'-acetoxylidide compound, a glycerol 1,2-bis-aminoalkyl ether compound, a benzisoxazole compound, an O-aminoalkylsalicylate compound, a heterocyclic phenoxyamine compound, a 2-substituted imidazo(1,2-A)pyridine compound, a 3-aryl substituted imidazo(1,2-A)pyridine compound, a polyorganophosphazene compound, a tertiary-alkylamino-lower acyl-xylidide compound, an amidinourea compound, a 3-(5'-adenylate) of a lincomycin compound, a 3-(5'-adenylate) of a clindamycin compound, an N-substituted derivative of a 1-(4'-alkylsulfonylphenyl)-2-amino-1,3-propanediol compound, a tertiary aminoalkoxyphenyl ether compound, an adenosine compound, adenosine, adenosine monophosphate, adenosine diphosphate, or adenosine triphosphate, a lauryl polyglycol ether compound, a 2-(.omega.-alkylaminoalkyl)-3-(4-substituted-benzylidene) phthalimidine compound, a 2-(.omega.-dialkylaminoalkyl)-3-(4-substituted-benzylidene)phthalimidine compound, an N,N,N-triethyl-N-alkyl ammonium salt, an L-N-n-propylpipecolic acid-2,6-xylidide compound, a polymer comprising repeating units of one or more local anesthetic moieties, an N-substituted 4-piperidinecarboxamide compound, an N-substituted 4-phenyl-4-piperidinecarboxamide compound; pharmaceutically acceptable derivatives thereof and combinations thereof.

In embodiments where the coating composition contains at least one anesthetic material, it is envisioned that any coating material suitable for application to the surface of a needle may be combined with the anesthetic material to form the coating composition used to coat the surgical needle. Some examples include, but are not limited to, lubricious coatings including silicones, siloxanes, polyorganosiloxanes, polydiorganosiloxanes, silanes, aminoalkyl siloxane and a cyclosiloxane, polydimethylsiloxane, and hydrocyclosiloxanes.

The coating composition containing at least one anesthetic material may be prepared utilizing a solvent. Any solvent known to those skilled in the art may used to form the coating compositions containing at least one anesthetic material and/or a coating material. Suitable examples include, but are not limited to, pentane, hexane, heptane, octane, etc., xylene, chlorinated solvents, THF, dioxanone, trichlorotrifluoroethane, 1,1,1-trichloroethane, alcohols, e.g., isopropyl alcohol and mineral spirits.

Once the coating composition containing at least one anesthetic material is formed, it can then be applied to the foregoing needles employing any suitable technique, e.g., by dipping, wiping, spraying, total immersion, etc, with dipping and spraying being the preferred techniques. For example, the needles are dipped into the coating mixture for a time period of from about 5 to about 60 seconds, typically from about 10 to about 45 seconds, in embodiments from about 15 to 30 seconds to form a coating on the needles. After evaporation of any dilutant or solvent carrier, the coated needles may be cured to the desired degree. Any suitable method of curing may be used to cure the coating on the needle, including, exposure to various amounts of heat, pressure, and humidity. In addition it is envisioned that the anesthetic coating need not be cured following the application process.

It will be understood that various modifications may be made to the embodiments disclosed herein. Therefore the above description should not be construed as limiting, but merely as exemplifications of preferred embodiments. For example, metal surfaces other than needles can be coated with the coating mixture in accordance with the methods described herein. Those skilled in the art will envision other modifications with the scope and spirit of the claims appended hereto.

## Claims

1. A method for manufacturing a coated surgical needle comprising applying to a surgical needle a coating composition comprising at least one anaesthetic material.

2. The method of Claim 1 wherein the at least one anaesthetic material is selected from ambucaine, amolanone, amylocaine, benoxinate, benzocaine, betoxycaine, biphenamine, bupivacaine, levo-bupivacaine, butacaine, butamben, butanilicicaine, butethamine, butoxycaine, carticaine, cocaethylene, cyclomethycaine, dibucaine, dimethisoquin, dimethocaine, diperodon, dyclonine, ecgonidine, ecgonine, ethyl aminobenzoate, ethyl chloride, levo-etidocaine, etidocaine, dextro-etidocaine, β-eucaine, euprocin, fenalcomine, fomocaine, hexylcaine, hydroxyprocaine, hydroxytetracaine, isobutyl p-aminobenzoate, leucinocaine mesylate, levoxadrol, meperidine, levo-mepivacaine, lidocaine, mepivacaine, meprylcaine, metabutoxycaine, methyl chloride, myrtecaine, naepaine, octacaine, orthocaine, oxethazaine, parethoxycaine, phenacaine, phenol, a pipecoloxylidide, piperocaine, piridocaine, polidocanol, pramoxine, sameridine, prilocaine, propanocaine, proparacaine, propipocaine, propoxycaine, pseudococaine, pyrrocaine, quinine urea, risocaine, ropivacaine, levo-ropivacaine, salicyl alcohol, tetracaine, tolycaine, trimecaine, veratridine, zolamine, a 2-alkyl-2-alkylamino-2',6'-acetoxylidide compound, a glycerol 1,2-bis-aminoalkyl ether compound, a benzisoxazole compound, an O-aminoalkylsalicylate compound, a heterocyclic phenoxyamine compound, a 2-substituted imidazo(1,2-A)pyridine compound, a 3-aryl substituted imidazo(1,2-A)pyridine compound, a polyorganophosphazene compound, a tertiary-alkylamino-lower acyl-xylidide compound, an amidinourea compound, a 3-(5'-adenylate) of a lincomycin compound, a 3-(5'-adenylate) of a clindamycin compound, an N-substituted derivative of a 1-(4'-alkylsulfonylphenyl)-2-amino-1,3-propanediol compound, a tertiary aminoalkoxyphenyl ether compound, an adenosine compound, adenosine, adenosine monophosphate, adenosine diphosphate, or adenosine triphosphate, a lauryl polyglycol ether compound, a 2-(ω-alkylaminoalkyl)-3-(4-substituted-benzylidene) phthalimidine compound, a 2-(ω-dialkylaminoalkyl)-3-(4-substituted-benzylidene)phthalimidine compound, an N,N,N-triethyl-N-alkyl ammonium salt, an L-N-(n-propyl)pipecolic acid-2,6-xylidide compound, a polymer comprising repeating units of one or more local anaesthetic moieties, an N-substituted 4-piperidinecarboxamide compound, an N-substituted 4-phenyl-4-piperidinecarboxamide compound; pharmaceutically acceptable derivatives thereof and combinations thereof.

3. The method of claim 1 or 2 wherein the anaesthetic material comprises lidocaine.

4. The method of claim 1 or 2 wherein the anaesthetic material comprises benzocaine.

5. The method of any of claims 1-4, wherein the coating composition comprises a material selected from silicones, siloxanes, polyorganosiloxanes, polydiorganosiloxanes, silanes, aminoalkyl siloxanes, cyclosiloxanes, polydimethylsiloxanes, hydrocyclosiloxanes, and combinations thereof.

6. The method of claim 5, wherein the coating composition comprises a silicone.

7. The method of claim 6, wherein the coating composition comprises at least one polydialkylsiloxane having a molecular weight sufficient to provide a viscosity of the coating mixture of at least 10,000 cp and at least one other siliconization material.

8. The method of claim 7, wherein the at least one polydialkylsiloxane having a molecular weight sufficient to provide a viscosity of the coating mixture of at least 10,000 cp is selected from polydimethylsiloxanes, polydiethylsiloxanes, polydipropylsiloxanes and polydibutylsiloxanes.

9. The method of claim 7 or 8, wherein the at least one other siliconization material is selected from a siliconization material containing an aminoalkyl siloxane and at least one other copolymerizable siloxane,and a silicone oil.

10. The method of any of claims 1-9, wherein the coating composition is applied to the surgical needle by dipping, wiping, spraying, or total immersion.

11. The method of any of claims 1-10, wherein the coating composition further comprises at least one solvent.

12. The method of claim 11, wherein the solvent is selected from a C₅₋₁₀-hydrocarbon solvent, xylene, chlorinated solvents, THF, dioxanone, mineral spirits, alcohols and mixtures thereof.

13. The method of claim 11 or 12, which further comprises the step of evaporating the solvent from the coating composition after application to the surgical needle.

14. The method of any of the preceding claims, which further comprises the step of curing the coating composition applied to the surgical needle.

15. Surgical needle having a coating comprising at least one anaesthetic material and being obtainable by the method of any of claims 1-14.
